Europäisches Patentamt

European Patent Office

Office européen des brevets

(11) Publication number: **0 336 341 B1**

# EUROPEAN PATENT SPECIFICATION

(45) Date of publication of patent specification: **22.06.94** (51) Int. Cl.5: **C07K 15/04**, A01N 63/04

(21) Application number: **89105818.2**

(22) Date of filing: **03.04.89**

(54) Baculovirus proteins and viral pesticides containing same.

(30) Priority: **06.04.88 US 178259**

(43) Date of publication of application:
**11.10.89 Bulletin 89/41**

(45) Publication of the grant of the patent:
**22.06.94 Bulletin 94/25**

(84) Designated Contracting States:
**DE GB NL**

(56) References cited:

VIROLOGY, vol. 167, no. 1, 1988, pages
242-250, Academic Press, Inc.; A.C.G. DERK-
SEN et al.: "Alteration of a Lepidopteran
peritrophic membrane by baculoviruses and
enhancement of viral infectivity"

CAN. ENTOMOL., vol. 120, no. 6, 1988, pages
575-580; R.P. JAOUES: "Field tests on control
of the imported cabbageworm (Lepidoptera:
Pieridae) and the cabbage looper (Lepidop-
tera: Noctuidae) by mixtures of microbial
and chemical insecticides"

JOURNAL OF ECONOMIC ENTOMOLOGY, vol.
76, no. 2, April 1983, pages 368-374; M.K.
SEARS et al.: "Utilization of action thresh-

olds for microbial and chemical control of
Lepidopterous pests (Lepidoptera: Noc-
tuidae, Pieridae) on cabbage"

(73) Proprietor: **BOYCE THOMPSON INSTITUTE
FOR PLANT RESEARCH
Tower Road
Ithaca New York 14853-1801(US)**

(72) Inventor: **Granados, Robert R.
5 Eagleshead
Ithaca New York 14859(US)**

(74) Representative: **Buchner, Otto, Dr. et al
Patentanwälte Dipl.-Ing. Klaus Westphal
Dr. rer. nat. Bernd Mussgnug
Dr. rer. nat. Otto Buchner
Flossmannstrasse 30a
D-81245 München (DE)**

EP 0 336 341 B1

**Description**

The invention relates to new baculovirus proteins, baculovirus pesticides containing them, their preparation, and use. More particularly, the invention relates to pest control compositions effective against insect pests and particularly against lepidopterous larvae comprising a nuclear polyhedrosis virus and a viral-coded protein factor which enhances infectivity and speed of kill.

The development and use of microbial agents as alternatives to chemicals for controlling noxious insect population has attracted increased attention and interest in recent years because of the public's increased awareness in maintaining the quality of the environment. The accumulation of pesticide residues in air, soil, water, and animals has helped to bring this heightened interest about. The insect pathogens in the family Baculoviridae, by virtue of their specificity, virulence, and safety for non-target species, have become logical candidates in this regard.

Several baculoviruses have been registered with the United States Environmental Protection Agency for use in the United States. Of the baculovirus products registered by the EPA, at least one, Elcar, the Heliothis zea nucleopolyhedrosis virus, was commercialized by Sandoz. Others which are registered for use under the auspices of the USDA Forest Service include Gypchek for control of the gypsy moth, Lymantria dispar, and TM-Bicontrol-1, for use against the Douglas-fir tussock moth, Orgyia pseudotsugata. A baculovirus product, Neochek S, has been used in Europe for control of the European pine sawfly, Neodiprion sertifer.

From Y.Tanada: A Synopsis of Studies on the Synergistic Property of an Insect Baculovirus: A Tribute to E.A.Steinhaus; J.Invertebrate Pathology, Vol.45, pp.125-138 (1985) there is known that a synergistic factor (SF) in the capsule matrix of a granulosis virus (GV) was found. The SF is a lipoprotein that occurs in the matrices of at least two granulosis viruses. It is coded by the viral genome. It enhances the infectivities of certain baculoviruses under in vivo and in vitro conditions. The phospholipid of the SF molecule is essential for the enhancing activity. The enhancement is associated with electric charges on the surfaces of the viral envelope and cell plasma membrane. The SF acts as an attachment molecule for receptor sites of the host cell plasma membrane and serves as a receptor molecule for the enveloped virion. The attachment sites of several baculoviruses onto SF are similar, and the receptor sites on cells of different insects are comparable.

T.Yamamoto, Y.Tanada: Phospholipid, an Enhancing Component in the Synergistic Factor of a Granulosis Virus of the Armyworm, Pseudaletia unipuncta; J.Invertebrate Pathology, Vol.31, pp.48-56 (1978) is describing the SF factor more specificly and can be summarized as follows: The synergistic factor (SF) in the capsule of a granulosis virus (Hawaiian strain) of the armyworm, Pseudaletia unipuncta, contains polypeptides and phospholipids. Its molecular weight is estimated by SDS-polyacrylamide gel electrophoresis as 126,000 +/- 8,700. The synergistic factor is rapidly depolymerized when phospholipase C is added. Phospholipase C alone does not decompose the synergistic factor, but it does destroy the capacity of the synergistic factor to enhance the nuclear polyderosis virus. In contrast, phospholipase $A_2$ has no effect on the synergistic factor.

The development of viral insecticides has been patterned after conventional pesticidal use and technology, and this, in turn, has led in part at least to less than expected results when viral insecticides are used as substitutes for chemical pesticides. There are many factors to consider for effective use of insecticides; the size and age of the insect population, the time of day, and the means of application. There is also an education problem. Farmers like to see insects die immediately after treatment, and unmodified baculovirus insecticides usually take 5-7 days to kill. Failure to bring pest population below the economic threshold along with lack of quickness of kill are two of the main deficiencies of viral pesticides.

The present invention overcomes some of the problems described above and satisfies all of the requirements for a safe, effective, and inexpensive insecticide by providing baculovirus pest control compositions having enhanced viral infectivity and speed of kill. Such compositions comprise a nucelar polyhedrosis virus, e.g. Autographa californica, and a protein purified from the granulin fraction of Trichoplusia ni granulosis virus occlusion bodies, from the granulin fraction of Heliothis armigera granulosis virus occlusion bodies or from the polyhedron fraction of nuclear polyhedrosis viruses. The invention embraces baculovirus coded proteins capable of degrading specific glycoproteins of the peritrophic membrane and destroying the structural integrity of this membrane in Trichoplusia ni larvae. These baculovirus enhancing proteins (subgroup B of genus Baculovirus) are characterized by molecular weights between 101 and 104 Kd.

Nuclear polyhedrosis viruses (family: Baculoviridae) are rod-shaped, enveloped particules containing a double-stranded, closed circular DNA genome. It is well-established that members of subgroup A of baculoviruses generate two distinct phenotypes which are involved in causing disease in susceptible

2

lepidopteran hosts such as the cabbage looper, Trichoplusia ni. The occluded virus form derives its envelope in the nucleus prior to occlusion in proteinaceous occlusion bodies. This is the primary phenotype responsible for the horizontal transmission of the virus in insect populations. The occluded viruses are released from the protein matrix upon contact with the alkaline midgut fluid of a feeding larva following ingestion of occlusion bodies. The virions released from the occlusion bodies infect midgut columnar cells and initiate the infection cycle.

Prior to the infection of midgut cells of a larval host, the virions released from occlusion bodies in the midgut lumen must survive the alkaline digestive fluids and pass through a peritrophic membrane which lines the midgut lumen. The peritrophic membrane is a noncellular tube comprised primarily of proteins, chitin, and glycosaminoglycans. It is generally nonporous to particles larger than 20 nm and is believed to serve as a barrier to invading microorganisms. With the occlusion bodies of Trichoplusia ni granulosis virus, Applicant has found at least two virus-coded proteins with enzymatic activity which degrade specific glycoproteins of the peritrophic membrane of Trichoplusia ni larvae, thereby changing the structure and presumably the permeability of the peritrophic membrane. Virus enhancing factors with similar characteristics have also been found in occlusion bodies of Autographa californica nuclear polyhedrosis virus and Heliothis armigera granulosis virus.

## VIRUS-ENHANCING PROTEIN FACTORS

To purify the T.ni granulosis virus occlusion bodies from infected larvae, the larvae were homogenized in water, filtered through 4 layers of cheesecloth, and the occlusion bodies were pelleted for 10 minutes at 8000 g for nuclear polyhedrosis viruses and 25 minutes at 12,000 g for granulosis viruses. After treatment with 1 % SDS (w/v) for 30 minutes at room temperature, the occlusion bodies were pelleted and washed three times in water.

$1.7 \times 10^{12}$ T.ni granulosis virus occlusion bodies were then dissolved in 1 ml 0.05 M sodium carbonate for 15 minutes at room temperature, and layered on a 20 % sucrose cushion in water and centrifuged for 45 minutes at 126,000 g at 4° C. The granulin fraction remained on top of the sucrose cushion and was collected. After an incubation of 5 hours at 28° C, the granulin fraction was applied onto a Sephacryl-S-200 Superfine (Pharmacia) column (2.6 x 34 cm) and eluted with 50 mM Tris-HCl pH 7.0, 0.1 M NaCl at 1.5 ml/min, and the absorption of the eluate measured at 280 nm. The fractions were collected and tested for the presence of enzymatic activity. The virus enhancing proteins in the fractions were analyzed on a sodium dodecyl sulfate (SDS) polyacrylamide gel. Protein concentrations of the fractions were determined.

## CHARACTERIZATION OF THE ENZYMATIC PROPERTIES OF THE VIRAL ENHANCING PROTEINS

The temperature optimum was determined by incubating the viral enhancing proteins and the peritrophic membrane at different temperatures for 5, 15 and 30 minutes, respectively. Enzyme-inactivating temperatures were determined by heat treatment of the viral enhancing protein for 30 minutes at 50°, 60°, 70°, and 80° C, and for 10 minutes at 95° C. The pH-optimum of the enzyme reaction was studied by using various dilutions of the viral enhancing proteins in buffers ranging in pH from 6.2 to 10.5. UV inactivation studies were performed by irradiating a viral enhancing protein solution up to $8.64 \times 10^6$ ergs/$cm^2$. The salt optimum was determined by adding sodium chloride to the reaction mixture at a final concentration of 0.1, 0.2, 0.3, 0.4, 0.5, 0.75, 1.0, 1.5 and 2.0 M. The following protease inhibitors were tested by addition to a viral enhancing protein-peritrophic membrane reaction mixture: beta-mercaptoethanol and dithiothreitol at 2.5, 10, 2O , 40, 80 and 160 mM final concentration; phenylmethenesulfonylfluoride and iodoacetate at final concentrations of 0.01, 0.5, 0.1, 0.5, 1, 2 and 5 mM. Ten $\mu$l of viral enhancing proteins were treated with 1 unit phospholipase $A_2$, C and D at pH 8.9, 7.3 and 5.6, respectively for 0.5, 1, 2, and 4 hours in an 11 $\mu$l incubation mixture. After phospholipase treatment, the pH of the reaction mixture was adjusted to 7.5 and 10 $\mu$l of treated viral enhancing proteins were tested. The control experiments were viral enhancing proteins treated with heat inactivated (15 min at 100° C) phospholipases, and heat inactivated (10 min at 100° C) viral enhancing proteins incubated with phospholipases. After incubation the viral enhancing proteins were analyzed on SDS polyacrylamide gels.

Using polyacrylamide gel electrophoresis the molecular weight of the viral enhancing proteins were determined on 7.5 % gels. The gels were silver stained and glyco-proteins were detected by periodic acid schiff (PAS) staining. For the 2 D gel electrophoresis the self-digested granulin (5 hours at 28° C) was separated on a native 7.5 % polyacrylamide gel. The gel was incubated in 2x lysis buffer (4 % SDS, 20 mM Tris-HCl pH 8.0, 2 mM ehtylenediaminatetraacetic acid (EDTA)), 20 % glycerol. Bromophenol Blue for 1/2 hour at room temperature and subsequently layered onto the 12.5 % SDS polyacrylamide gel. After electrophoresis, the proteins were visualized by silver staining. The 101 and 104 K proteins were extracted from a native gel by crushing a gel slice containing both proteins in 1 ml water and incubating for 5 hours at room temperature. The gel pieces were removed by filtering over siliconized glass wool and the sample was dialyzed against water overnight at 4° C. After lyophilization, the proteins were dissolved in 50 mM Tris-HCl pH 7.0, 0.1 M NaCl. This sample was then tested for enzyme activity. Controls consisted of gel pieces removed from a different area of the lane. The sample containing the two proteins with molecular weights of 101 and 104 Kd exhibited enzyme activity.

The 101 and 104 Kd proteins migrated as one band in a 7.5 % native gel. This was confirmed by purification of the proteins from the high molecular weight band from the native gel. The 12.5 % SDS polyacrylamide gel showed that the eluant contained purified 101 and 104 Kd proteins. The 2D-gel electrophoresis demonstrated that the proteins were not composed of subunits.

The characteristics of the virus enhancing proteins of Trichoplusa ni granulosis virus are summarized in TABLE 1.

Based on the results of a larval bioassay, the proteins were ten times more stable to UV irradiation than the occluded virions. Whereas a dose of $3 \times 10^5$ ergs/cm$^2$ resulted in 93 % inactivation of occlusion body infectivity, $3 \times 10^6$ ergs/cm$^2$ was necessary to inactivate the enzyme activity.

THE EFFECT OF THE GRANULIN FRACTION (101/104 Kd PROTEINS) AND THE POLYHEDRIN FRACTION ON THE INFECTIVITY OF AUTOGRAPHA CALIFORNICA NUCLEAR POLYHEDROSIS VIRUS IN THE 5TH INSTAR TRICHOPLUSIA NI LARVAE

The results of three bioassays, conducted with 20 to 30 larvae at each dose, are summarized in TABLE 2. The $LD_{50}$ and $LD_{90}$ for the virus treated larvae were 9 and 141 occlusion bodies, respectively. The mortalities, recorded with the active granulin was added to Autographa californica nuclear polyhedrosis virus occlusion bodies, indicated that receipt of one occlusion body was enough to kill fifth instar larvae. Thus, roughly a greater than 25-fold increase in infectivity was realized by the addition of the granulin fraction containing the 101/104 K proteins to the occlusion bodies, even when it was 10 to 100 times diluted. The increase in infectivity which may be realized by the addition of the proteins found in the polyhedrin fraction of AcMNPV or the granulin fraction of HaGV is less than 5 fold.

TABLE 1

| Characteristics of the virus enhancing proteins of Trichoplusia ni granulosis virus | |
|---|---|
| Molecular weight:<br>pH optimum:<br>Temperature optimum:<br>Heat inactivation temperature:<br>UV inactivation:<br>Salt preference: | 101 and 104 Kd proteins without subunits<br>pH 8<br>50° C<br>30 min 80° C<br>$3 \times 10^6$ ergs/cm$^2$<br>0.2-2.0 M NaCl |
| Inhibited with | 40 mM $\beta$-mercaptoethanol<br>5 mM dithiothreitol<br>1 mM iodoacetate |
| Not inhibited by 5 mM phenylmethenesulfonylfluoride<br>Resistant against alkaline proteases of T.ni larvae<br>Not stained by glycoprotein staining (PAS)<br>Not inactivated or degraded by phospholipase A$_2$, C or D | |

4

## TABLE 2

Bioassay of various concentrations of *Autographa californica* occlusion bodies in the presence of the granulin fraction from *Trichoplusia ni* GV at different dilutions, fed to fifth instar *Trichoplusia ni* larvae

### % mortality in the presence of

| Expt. | Occlusion Bodies/ larvae | heat inact.[a] gran.fr. | gran. fr.[b] | $10^{-1}$ gran.fr. | $10^{-2}$ gran.fr. | $10^{-3}$ gran.fr. |
|---|---|---|---|---|---|---|
| Control[c] | 0 | | 0 | 0 | | |
| 1[d] | 1.12 | 14 | | 73 | | |
| 2 | 5.6 | 45 | 90 | | | 50 |
| 3 | 14 | 61 | 100 | 100 | 80 | |
| 3 | 28 | 64 | | | | |
| 3 | 140 | 100 | | | | |

a/ Granulin fractions were heat inactivated for 10 min. at 100° C.

b/ The amount of granulin in the undiluted sample was comparable with the granulin that can be released from 1.5 x $10^9$ *Trichoplusia ni* granulosis virus.

c/ Control treatments consisting of heat inactivated granulin gave 0 % mortality in all experiments.

d/ The granulin fraction used in experiment 1 was concentrated and filtered through an Amicon filter with 50 K molecular weight cutoff. Aliquots of granulin fractions from experiments 1 and 2 were tested in an *in vitro* peritrophic membrane assay and both samples showed peritrophic membrane glycoprotein-degrading activity.

The baculovirus proteins of the present invention are useful as components of pesticides. They enhance the infectivity of viral pesticides, especially *Autographa californica* nucleus polyhedrosis virus. Viral pesticides containing the novel proteins of the present invention can be mixed with any of a variety of biological pesticides including *Bacillus thuringiensis*, B.T., as well as with chemical pesticides such as Sevin.

The viral insecticides containing the baculovirus proteins of this invention can be applied in any of a variety of ways heretofore used in integrating baculoviruses into pest management strategies. For example, the direct control of outbreak populations of insects involves broadcast application, either from aircraft or with spray equipment. Aerial application is especially useful in viral control of forest pests. For ground application foggers and mistblowers may be used. Other tactics which may be employed include the release of both virus infected and contaminated hosts and the mechanical manipulation of the environment to make the baculovirus more available for host comsumption. It is to be understood that the choice of tactical approaches in using baculoviruses as pesticides depends on the dynamics of the host-pest system to be managed and the relative threat of economic damage. It may be possible to intercede with spot inoculation tactics early in the insect's developmental cycle, or in the preceding generation. However, waiting until pest numbers have reached the economic threshold almost certainly will require the use of broadcast application.

**Claims**

1. Baculovirus proteins free of occlusion bodies which proteins break down the physical structure of the perithrophic membrane of insects having a peritrophic membrane.

2. Baculovirus proteins free of occlusion bodies which proteins break down the physical structure of the perithrophic membrane of lepidopterous larvae.

3. Baculovirus proteins free of occlusion bodies and all viral particles other than those which degrade structural glycoproteins of the peritrophic membrane of Trichoplusia ni larvae, said proteins being characterized by having molecular weights of about 101 and about 104 Kd and being resistant to the midgut alkaline proteases of T.ni larvae.

4. A polyhedrin fraction isolated from a nuclear polyhedrosis virus and free from occlusion bodies and other viral particles, said fraction being characterized by enhancing the infectivity of nuclear polyhedrosis viruses against insect species.

5. Baculovirus proteins of claim 2 present in the granulin fraction of the Trichoplusia ni granulosis virus.

6. Baculovirus proteins of claim 2 present in the granulin fraction of the Heliothis armigera granulosis virus.

7. Baculovirus proteins of claim 2 present in the polyhedron fraction of Autographa californica nuclear polyhedrosis.

8. Proteins which are obtained from Trichoplusia ni granulosis virus and characterized by the following properties: Momeric molecular weights of about 101 and about 104 Kd, pH optimum of 8, temperature optimum $50°C$, heat inactivation 30 min. $80°C$, UV inactivation 3 X 10 ergs/$cm_2$, salt preference 0.2-2.0 M NaCl, stable in 4 M urea; inhibited with 40 mM $\beta$-mercaptoethanol, 5 mM dithiothreitol und 1 mM iodoacetate; not inhibited by 5 mM phenylmethenesulfonylfluoride; resistant against alkaline midgut proteases of Trichoplusia ni larvae; not stained by glycoprotein staining; and not inactivated or degraded by phospholipase $A_2$, C or D.

9. A viral pesticide comprising a nuclear polyhedrosis virus and a viral factor which enhances infectivity, said factor comprising a protein of claim 1.

10. A viral pesticide comprising Autographa californica nuclear polyhedrosis virus and a viral factor which enhances infectivity, said factor comprising proteins with molecular weights of about 101 and about 104 Kd present in the granulin fraction of the Trichoplusia ni granulosis virus.

11. A process of obtaining the baculovirus proteins of claim 1 which comprises the steps of sedimentation of the Trichoplusia ni granulosis virus particles, dissolving the virus occlusion bodies in sodium carbonate, high speed centrifugation to remove virus particles, incubating the granulin fraction for 5 hours at room temperature, applying the granulin fraction onto a Sephacryl column, and eluting the column with a Tris buffer to collect active protein enhancing fractions.

**12.** A mixture for controlling insect pests having peritrophic membranes comprising a biological pesticide and (a) a polyhedrin fraction of claim 4 or (b) a protein of claim 1.

**13.** A mixture for controlling insect pests comprising a chemical pesticide an a viral pesticide of claim 9.

**14.** Mixture of claim 12, wherein the biological pesticide is the toxin protein produced by the bacterium Bacillus thuringiensis.

**15.** A method for controlling insect pests comprising applying to the pest a chemical pesticide and a viral pesticide of claim 9.

**16.** A process for enhancing Autographa californica nuclear polyhedrosis virus infection in larvae of Trichoplusia ni which involves incorporating the viral enhancing fraction of claim 4 into the Autographa california nuclear polyhedrosis virus inoculum.

## Patentansprüche

**1.** Proteine des Baculovirus, die frei von Okklusionskörpern sind, und die die physikalische Struktur der peritrophen Membran von Insekten mit einer peritrophen Membran auflösen.

**2.** Proteine des Baculovirus, die frei von Okklusionskörpern sind, und die die physikalische Struktur der peritrophen Membran der lepidopteren Larven auflösen.

**3.** Proteine des Baculovirus, die frei von Okklusionskörpern und von allen viralen Partikeln sind, mit Ausnahme derjenigen, die die strukturellen Glycoproteine der peritrophen Membran der Trichoplusia ni-Larven abbauen, dadurch gekennzeichnet, daß sie ein Molekulargewicht von etwa 101 und etwa 104 Kd aufweisen und gegenüber den alkalischen Proteasen des mittleren Teils des Verdauungskanals der T.ni-Larven resistent sind.

**4.** Polyhedrinfraktion, die aus einem Kernpolyhedrosevirus isoliert wurde, und die frei von Okklusionskörpern und weiteren viralen Partikeln ist, dadurch gekennzeichnet, daß sie die Ansteckungsfähigkeit der Kernpolyhedroseviren gegen Insektenspezien steigert.

**5.** Proteine des Baculovirus nach Anspruch 2, die in der Granulinfraktion des Trichoplusia ni-Granulosevirus vorliegen.

**6.** Proteine des Baculovirus nach Anspruch 2, die in der Granulinfraktion des Heliothis armigera-Granulosevirus vorliegen.

**7.** Proteine des Baculovirus nach Anspruch 2, die in der Polyhedronfraktion der Autographa californica-Kernpolyhedrose vorliegen.

**8.** Proteine, die man aus einem Trichoplusia ni-Granulosevirus erhält und die durch die folgenden Eigenschaften charakterisiert sind: Monomere (momeric) Molekulargewichte von etwa 101 und etwa 104 Kd, pH-Optimum von 8, Temperaturoptimum 50°C, Hitzeinaktivierung 30 Minuten bei 80°C, UV-Inaktivierung 3 x 10 Erg/cm$^2$, Salzpräferenz 0,2-2,0 M NaCl, stabil in 4 M Harnstoff; mit 40 mM $\beta$-Mercaptoethanol, 5 mM Dithiothreitol und 1 mM Iodacetat inhibiert; nicht inhibiert durch 5 mM Phenylmethylensulfonylfluorid; resistent gegen alkalische Proteasen des mittleren Teils des Verdauungskanals von Trichoplusia ni-Larven; nicht gefärbt durch Glycoproteinfärbung sowie keine Inaktivierung oder Abbau durch Phospholipase A$_2$, C oder D.

**9.** Virales Pestizid, das ein Kernpolyhedrosevirus und einen viralen Faktor enthält, der die Ansteckungsfähigkeit steigert, wobei der Faktor ein Protein nach Anspruch 1 enthält.

**10.** Virales Pestizid, das ein Autographa californica-Kernpolyhedrosevirus und einen viralen Faktor enthält, der die Ansteckungsfähigkeit steigert, wobei der Faktor Proteine mit Molekulargewichten von etwa 101 und etwa 104 Kd enthält, die in der Granulinfraktion des Trichoplusia ni-Granulosevirus vorliegen.

**11.** Verfahren zum Erhalt von Proteinen des Baculovirus nach Anspruch 1, das die folgenden Schritte umfaßt:
Sedimentierung der Partikel des Trichoplusia ni-Granulosevirus, Auflösen der Virusokklusionskörper in Natriumcarbonat, Hochgeschwindigkeitszentrifugieren um Viruspartikel zu entfernen, Inkubieren der Granulinfraktion 5 Stunden lang bei Raumtemperatur, Aufgeben der Granulinfraktion auf eine Sephacrylkolonne und Eluieren der Kolonne mit einem Trispuffer, um Fraktionen zu sammeln, die aktives Protein steigern.

**12.** Mischung zur Bekämpfung von Insektenschädlingen mit peritrophen Membranen, die ein biologisches Pestizid und (a) eine Polyhedrinfraktion nach Anspruch 4 oder (b) ein Protein nach Anspruch 1 enthält.

**13.** Mischung zur Bekämpfung von Insektenschädlingen, die ein chemisches Pestizid und ein virales Pestizid nach Anspruch 9 enthält.

**14.** Mischung nach Anspruch 12, bei der das biologische Pestizid das Toxinprotein darstellt, das von dem Bakterium Bacillus thuringiensis produziert wird.

**15.** Verfahren zur Bekämpfung von Insektenschädlingen, das darin besteht, ein chemisches Pestizid und ein virales Pestizid nach Anspruch 9 auf den Schädling aufzubringen.

**16.** Verfahren zur Steigerung der Infektion des Autographa californica-Kernpolyhedrosevirus in Larven der Trichoplusia ni, das die Aufnahme der viralen steigernden Fraktion des Anspruchs 4 in das Autographa californica-Kernpolyhedrosevirus-Impfmaterial einschließt.

**Revendications**

**1.** Protéines de baculovirus libres de corps d'occlusion, ces protéines décomposant la structure physique de la membrane péritrophique d'insectes comportant une membrane péritrophique.

**2.** Protéines de baculovirus libres de corps d'occlusion, ces protéines décomposant la structure physique de la membrane péritrophique de larves de lépidoptères.

**3.** Protéines de baculovirus libres de corps d'occlusion et de toutes particules virales autres que celles qui dégradent les glycoprotéines de structure de la membrane péritrophique de larves de Trichoplusia ni, ces protéines étant caractérisées en ce qu'elles présentent des poids moléculaires environ de 101 à 104 Kd, et sont résistantes aux protéases alcalines de l'intestin moyen de laves de T. ni.

**4.** Fraction de polyhédrine isolée à partir d'un virus polyhédrosis nucléaire et libre de corps d'occlusion et autres particules virales, cette fraction étant caractérisée en ce qu'elle renforce la contagion de virus polyhédrosis nucléaires contre des espèces d'insectes.

**5.** Protéines de baculovirus selon la revendication 2, présentes dans la fraction granuline du virus Trichoplusia ni granulosis.

**6.** Protéines de baculovirus selon la revendication 2, présentes dans la fraction granuline du virus Heliothis armigera granulosis.

**7.** Protéines de baculovirus selon la revendication 2, présentes dans la fraction de polyhedrosis Autographa Californica nucléaire.

**8.** Protéines obtenues à partir du virus Trichoplusia ni granulosis et caractérisées par les propriétés suivantes : poids moléculaires momères environ de 101 et environ de 104 Kd, pH optimum de 8, température optimum 50°C, inactivation thermique 30 min. 80°C, inactivation UV 3 x 10 ergs/cm$^2$, préférence saline 0,2 - 2,0 M de NaCl, stable dans 4 M d'urée ; inhibées par 40 mM de $\beta$-mercaptoéthanol, 5 mM de dithiothreitol et 1 mM d'iodoacétate ; non inhibées par 5 mM de phénylmethenesulfonylfluorure ; résistantes aux protéases alcalines d'intestin moyen de larves de Trichoplusia ni ; non tachées par de la teinture de glycoprotéines ; et ni activées ni dégradées par du phospholipase A$_2$, C ou D.

9. Pesticide viral comprenant un virus polyhédrosis nucléaire et un facteur viral qui renforce la contagion, ce facteur comprenant une protéine de la revendication 1.

10. Pesticide viral comprenant le virus polyhédrosis nucléaire Autographa Californica et un facteur viral qui renforce la contagion, ce facteur comprenant des protéines présentant des poids moléculaires environ de 101 et environ de 104 Kd, présentes dans la fraction granuline du virus Trichoplusia ni granulosis.

11. Procédé d'obtention de protéines de baculovirus de la revendication 1, comprenant les étapes de sédimentation des particules de virus Trichoplusia ni granulosis, de dissolution des corps d'occlusion du virus dans du carbonate de sodium, de centrifugation à grande vitesse pour retirer les particules de virus, d'incubation de la fraction granuline pendant 5 heures à la température ambiante, d'application de la fraction granuline sur une colonne Sephacryl, et l'élution de la colonne par un tampon Tris pour collecter les fractions de renforcement de protéines actives.

12. Mélange pour enrayer les invasions d'insectes comportant des membranes péritrophiques, comprenant un pesticide biologique et (a) une fraction polyhédrine de la revendication 4 ou (b) une protéine de la revendication 1.

13. Mélange pour enrayer les invasions d'insectes, comprenant un pesticide chimique et un pesticide viral de la revendication 9.

14. Mélange de la revendication 12, dans lequel le pesticide biologique est la protéine de toxine produite par le bactérium Bacillus Thuringiensis.

15. Procédé pour enrayer les invasions d'insectes, consistant à appliquer à l'invasion un pesticide chimique et un pesticide viral de la revendication 9.

16. Procédé pour renforcer l'infection de virus Autographa californica polyhédrosis nucléaires dans des larves de Trichoplusia ni ; consistant à incorporer la fraction de renforcement virale de la revendication 4 dans le virus d'inoculation d'Autographa californica polyhédrosis nucléaire.